# EUROPÄISCHE PATENTSCHRIFT

(11) **EP 2 990 647 B1**
(45) Veröffentlichungstag und Bekanntmachung des Hinweises auf die Patenterteilung: **05.06.2019**
(21) Anmeldenummer: 15180899.5
(22) Anmeldetag: 13.08.2015
(51) Int. Cl.: F04B 43/12, F04B 45/08, A61M 5/142

(54) **SCHLAUCHPUMPE**
HOSE PUMP
POMPE TUBULAIRE

(30) Priorität: 27.08.2014 DE 102014112324
(43) Veröffentlichungstag der Anmeldung: 02.03.2016
(73) Patentinhaber: Stockert GmbH, 79111 Freiburg (DE)
(72) Erfinder: Höfling, Uwe, 77955 Ettenheim (DE)
(74) Vertreter: Lemcke, Brommer & Partner Patentanwälte Partnerschaft mbB

(56) Entgegenhaltungen:
- DE-A1- 3 943 430
- DE-A1- 19 717 452
- DE-U1-202012 103 619
- US-A- 4 564 342
- US-A1- 2012 020 822
- US-B2- 8 393 880

## Beschreibung

Die Erfindung betrifft eine Schlauchpumpe nach dem Oberbegriff des Anspruchs 1. Eine solche Schlauchpumpe enthält einen elastisch verformbaren Schlauch für ein zu förderndes Medium, ein Schlauchbett, über das der Schlauch geführt ist, sowie mindestens ein Quetschelement, das entlang des Schlauchbetts bewegbar ist. Das Quetschelement verformt den Schlauch auf dem Schlauchbett und fördert das Medium innerhalb des Schlauchs vorwärts, wenn es sich entlang des Schlauchbetts bewegt; die sich mit dem Quetschelement entlang des Schlauchbetts bewegende Verformungsstelle des Schlauchs, die den Schlauch zumeist vollständig abklemmt, treibt das zu fördernde Medium innerhalb des Schlauchs vorwärts. Ein pumpeneingangsseitiger Unterdruck im Schlauch wird normalerweise durch dessen elastische Rückstellkraft erzeugt, mittels der er sich hinter dem Quetschelement wieder in seine Ursprungsform zurück verformt.

Schlauchpumpen der vorliegenden Art werden auch als Peristaltikpumpen sowie zuweilen als Schlauchquetschpumpen bezeichnet. Sie werden in der Regel dort eingesetzt, wo geringe Fördermengen eines Mediums bei hoher Dosiergenauigkeit gefördert werden müssen, oder wo ein Medium gefördert wird, das wegen seiner chemischen Aggressivität, einer radioaktiven Kontaminierung oder insbesondere deswegen nicht in Kontakt mit sich bewegenden Pumpenelementen in Berührung kommen soll, weil es chemisch hochrein ist oder medizinischen Zwecken dient. Dementsprechend werden Schlauchpumpen der vorliegenden Art bevorzugt im Bereich der Medizintechnik eingesetzt, in welchem deren Vorteile besonders zum Tragen kommen; beispielsweise bei einer Infusion von Kochsalzlösung mit oder ohne pharmazeutisch wirksame Inhaltsstoffe, von Kontrastmitteln und dergleichen mehr.

In der einfachsten Bauform weist eine Schlauchpumpe der vorliegenden Art einen Pumpenkopf mit einer in etwa halbkreisförmig ausgebildeten Innenkontur auf. In der Innenkontur des Pumpenkopfs ist das Schlauchbett angeordnet, in welches der Schlauch eingelegt ist. Ein innerhalb des Halbkreises angeordnetes Pumpenrad trägt meist zwei oder mehr Quetschelemente in Form von Rollen, die bei einer Rotation des Pumpenrads auf dem Schlauch abrollen und diesen hierbei gegen das Schlauchbett quetschen. Das Pumpenrad wird von einem elektronisch gesteuerten Antrieb bewegt, wobei die elektronische Steuerung die Dosierung des mit der Schlauchpumpe geförderten Mediums regelt. Solche Schlauchpumpen sind beispielsweise aus der DE 20 2012 103 619 U1, der DE 20 2010 000 282 U1 oder der DE 20 2012 009 626 U1 bekannt.

Weitere Beispiele für Schlauchpumpen sind in der DE 197 17 452 A1, der US 2012/0020822 A1 und der US 8,393,880 B2 offenbart. Bei der erstgenannten und der letztgenannten dieser drei Schriften handelt es sich um alternative Konstruktionen, bei denen auf einer Scheibe ein mit einer Membran abgedeckter Pumpkanal oder eine Auflagefläche für einen Schlauch vorgesehen ist und ein Quetschelement den Schlauch gegen die Scheibe bzw. die Membran in den Pumpkanal drückt. Im letztgenannten Dokument ist alternativ vorgesehen, einen Schlauch beidseits mit Quetschelementen in Form von Rollen zu Quetschen, um eine Förderbewegung zu erzeugen. Die US 2012/0020822 A1 weist ebenso entweder einen Pumpkanal auf, der durch eine Membran abgedeckt ist, welche vom Quetschelement in den Pumpkanal hineingedrückt wird, um die Förderbewegung zu erzeugen, oder aber ein speziell geformter Schlauch liegt auf einem Schlauchbett auf und wird mit Quetschelementen in Form von Rollen zum Erzeugen der Förderbewegung gequetscht.

Ein bekanntes Problem bei Schlauchpumpen der vorliegenden Art ist die starke mechanische Beanspruchung des Schlauches. Denn um den notwendigen Förderdruck zu erzeugen, muss der Schlauch stark gequetscht werden, was dessen Lebensdauer stark beeinträchtigt. In der bereits erwähnten DE 20 2012 103 619 U1 sowie in der DE 20 2013 100 124 U1 wird zur Lösung dieser Problematik vorgeschlagen, mehrlagige Schläuche zu verwenden, wobei in der letztgenannten Schrift zum Stand der Technik auch die Verwendung von mechanisch nachgiebigen Quetschelementen und/oder eines mechanisch nachgiebigen Schlauchbetts vorgeschlagen wird.

Eine weitere Problematik bei der Verwendung von Schlauchpumpen in der Medizintechnik, und dort insbesondere bei Schlauchpumpen, die über einen Katheter mit dem Gefäßsystem eines Patienten verbunden sind, besteht im Erzeugen von Druckpulsen und von geringen Ladungstrennungen beim Verformen des Schlauchs. Denn die beim Pumpen erzeugten Ladungstrennungen sowie die mechanischen Impulse, die über den Schlauch und den Katheter in das Gefäßsystem des Patienten eingeleitet werden, können unter Umständen laufende EKG-Messungen stören, und zwar zum einen aufgrund der elektrischen Ladungen, die das bei EKG-Messungen sehr hochohmig und mit sehr hoher Genauigkeit ermittelte elektrische Signal überlagern und die vom Arzt ausgewertete Signalform des EKG-Signals verändern können, sowie zum anderen durch die mechanischen Impulse, deren Frequenz in der Größenordnung des vom EKG gemessenen Herzschlags liegen und hierdurch die Messung beeinträchtigen oder gar verfälschen können.

Der vorliegenden Erfindung liegt daher die Aufgabe zugrunde, eine Schlauchpumpe der eingangs genannten Art insbesondere für den Einsatz in medizintechnischen Anwendungen hinsichtlich ihres Störpotentials für EKG-Messungen zu optimieren sowie für alle Anwendungen hinsichtlich der Lebensdauer des Schlauchs zu verbessern.

Gelöst ist diese Aufgabe durch eine Schlauchpumpe mit den Merkmalen des Anspruchs 1. Bevorzugte Ausgestaltungen und Weiterbildungen der erfindungsgemäßen Schlauchpumpe sind in den Ansprüchen 2 bis 8 niedergelegt.

Eine Schlauchpumpe der eingangs genannten Art ist also erfindungsgemäß zunächst dadurch modifiziert, dass das Schlauchbett zumindest zum Teil mit einer reibungsvermindernden Beschichtung versehen ist, und zwar bevorzugt auf einer Lauffläche des Schlauchs. Diese reibungsvermindernde Beschichtung haftet vorzugsweise fest am Schlauchbett an und kann beispielsweise im Wesentlichen aus Kunststoffen, wie insbesondere PTFE (Polytetrafluorethylen) oder Parylenen und/oder aus Graphit, Diamant-Graphit und/oder einem harten Fett bestehen.

Erfindungsgemäß ist erkannt worden, dass durch die Friktion zwischen dem Schlauch und dem Schlauchbett ein verhältnismäßig starkes Walken des Schlauchs beim Verformen auftritt und dieses Walken zu unnötigen und ungewollten Quetschungen im Schlauchkörper führt. Die erfindungsgemäße reibungsvermindernde Beschichtung des Schlauchbetts ermöglicht geringe Ausweichbewegungen des Schlauchs auf dem Schlauchbett, wodurch er schonender verformt wird und die unerwünschten Quetschungen vermieden werden. Dies erhöht naturgemäß die Lebensdauer des Schlauches.

Darüber hinaus ist erfindungsgemäß erkannt worden, dass die schonendere Verformung des Schlauchs auf einem mit einer reibungsvermindernden Beschichtung versehenen Schlauchbett auch geeignet ist, nachteilige Druckpulse signifikant zu vermindern und die beim Verformen von vielen üblichen Schlauchmaterialien entstehenden Ladungstrennungen zu verringern. Nach Beobachtungen der Anmelderin hängt dies damit zusammen, dass der Schlauch auf dem erfindungsgemäßen Schlauchbett durch das Quetschelement nicht so abrupt verformt wird, wie dies im Stand der Technik der Fall ist.

Im Rahmen der vorliegenden Erfindung hat es sich als weiterhin vorteilhaft herausgestellt, wenn ein Schlauchbett mit einem erfindungsgemäß modifizierten Profil verwendet wird. Dieses Profil ist bezüglich einer in Längsrichtung des Schlauchs verlaufenden Mittellinie symmetrisch oder asymmetrisch konkav geformt, wobei ein die Mittellinie enthaltender mittlerer Bereich einen ersten Krümmungsradius aufweist und zwei an den mittleren Bereich angrenzende Nachbarbereiche zweite Krümmungsradien aufweisen, die größer als der erste Krümmungsradius sind. Zweckmäßigerweise ist der Übergang zwischen den Krümmungsradien stetig ausgebildet. Das Schlauchbett läuft also neben seinem mittleren Bereich etwas flacher aus, was sich auf die gewollt sanfte Verformung des Schlauchs bei der Förderbewegung des Quetschelements vorteilhaft auswirkt. Im Zusammenhang mit der erfindungsgemäßen reibungsvermindernden Beschichtung des Schlauchbetts kann der Schlauch sich hierdurch schonend verformen.

Die erfindungsgemäße Schlauchpumpe ist somit insbesondere für den Einsatz in der Medizintechnik, aufgrund ihres signifikant geringeren Störpotentials bei EKG-Messungen, gegenüber dem Stand der Technik deutlich verbessert. Darüber hinaus erhöht sich die Lebensdauer des in der Schlauchpumpe verwendeten Schlauchs, und zu guter Letzt ist auch die Geräuschentwicklung der Schlauchpumpe vorteilhaft vermindert.

Ganz besondere Vorteile bietet die vorliegende Erfindung, wenn die erfindungsgemäße Schlauchpumpe mit einem Herzkatheter verbunden ist, da die durch das Verformen des Schlauchs erzeugten Druckpulse bzw. Mikroimpulse sowie gegebenenfalls erzeugte Ladungstrennungspulse, so gering sie auch sein mögen, direkt in das Herz des Patienten geleitet werden, wo sie die laufende EKG-Messung signifikant beeinträchtigen können.

Überraschend hat sich herausgestellt, dass es weitere Vorteile bietet, wenn das Profil des Schlauchbetts sich nicht etwa nach außen hin noch weiter abflacht, sondern wenn das Profil zwei Randbereiche aufweist, die wiederum einen kleineren Krümmungsradius aufweisen als die abgeflachten Nachbarbereiche des mittleren Bereichs. Dies stützt den Schlauch beim Verformen. Bevorzugt laufen die Randbereiche des Profils mit einer konvexen Krümmung nach außen aus, so dass auch an dieser Stelle keine unerwünschten Klemmungen und Quetschungen des Schlauchs entstehen.

Im Rahmen der vorliegenden Erfindung ist es bevorzugt, als Quetschelemente Rollen zu verwenden, die auf dem Schlauch abrollen und diesen gegen das Schlauchbett verformen. Gegebenenfalls können diese Rollen dem Profil des Schlauchbetts angepasst sein.

Das Quetschelement ist vorzugsweise solcherart relativ zum Schlauchbett geführt, dass es bei einer Bewegung entlang des Schlauchbetts den Schlauch zunächst nur wenig verformt und mit zunehmendem Bewegungsweg stetig zunehmend bis zum vollständigen Abklemmen verformt. Hierdurch erfolgt die Verformung des Schlauchs besonders sanft und allmählich, was insbesondere zur Verminderung der unerwünschten Druckpulse nochmals zusätzlich beiträgt. Wie an sich bekannt, kann auch im Rahmen der vorliegenden Erfindung der Schlauch aus PVC, PTFE oder Silikon gefertigt sein, was insbesondere für medizintechnische Anwendungen Vorteile bietet.

Ein Ausführungsbeispiel für eine erfindungsgemäße Schlauchpumpe wird unter Verweis der beigefügten Zeichnungen im Folgenden näher beschrieben und erläutert. Es zeigen:
- Figur 1: eine schematische Schnittdarstellung von funktionswesentlichen Teilen einer erfindungsgemäßen Schlauchpumpe;
- Figur 2: eine perspektivische Darstellung eines Teils des Schlauchbetts aus Figur 1;
- Figur 3: eine Seitenansicht des Schlauchbetts aus Figur 2;
- Figur 4: ein Diagramm von gemessenen elektrischen Spannungen im Schlauch mit einem Schlauchbett nach dem Stand der Technik;
- Figur 5: ein Diagramm von gemessenen elektrischen Spannungen im Schlauch mit einem erfindungsgemäßen Schlauchbett.
- Figur 6: ein Diagramm zur Darstellung der Quetschung des Schlauchs auf dem Schlauchbett.

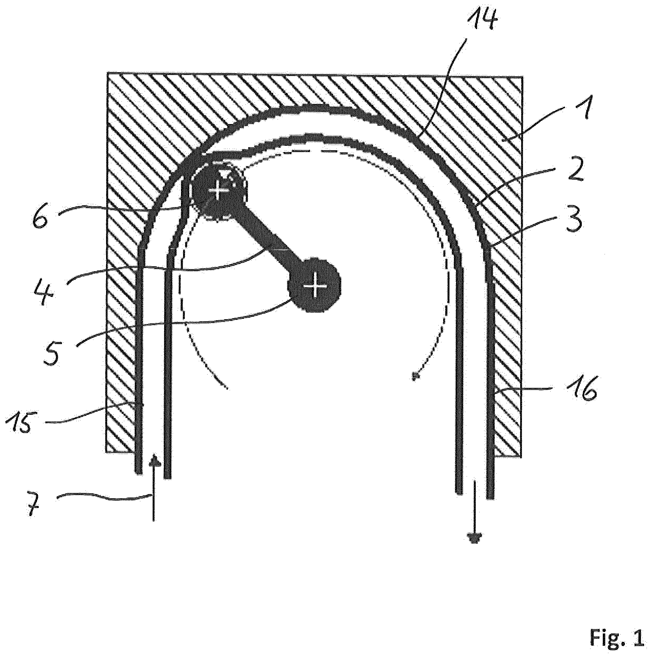

Figur 1 zeigt eine schematische Schnittdarstellung der funktionswesentlichen Teile einer erfindungsgemäß ausgestalteten Schlauchpumpe. Ein Pumpenkopf 1 mit einer halbkreisförmigen Innenkontur 14 trägt an dieser Innenkontur 14 ein Schlauchbett 2, in das ein Schlauch 3 eingelegt ist. Die halbkreisförmige Innenkontur 14 des Pumpenkopfs 1 setzt sich in einen geraden Einlaufabschnitt 15 und einen geraden Auslaufabschnitt 16 fort. Innerhalb der halbkreisförmigen Innenkontur 14 sitzt ein Pumpenrad 4 auf einer Antriebswelle 5, die (nicht dargestellt) von einem elektronisch gesteuerten Antriebsmotor angetrieben wird. Auf dem Pumpenrad 4 sitzt ein Quetschelement 6, das vorliegend als drehbar gelagerte Rolle ausgebildet ist.

Durch eine Drehung des Pumpenrads 4 im Uhrzeigersinn rollt das Quetschelement 6 auf dem Schlauch 3 ab und drückt diesen gegen das Pumpenbett 2, so dass sich der Schlauch 3 um das Quetschelement 6 herum verformt. Entlang der halbkreisförmigen Innenkontur 14 des Pumpenkopfs 1 wird der Schlauch 3 vom Quetschelement 6 vollständig abgeklemmt, wodurch ein im Schlauch 3 befindliches Medium 7 vorwärts geschoben wird. Auf der Rückseite der Verformungsstelle kehrt der Schlauch 3 in seine Ursprungsform zurück, wodurch ein Unterdrück für das zu fördernde Medium 7 entsteht.

Durch die kreisförmige Bewegung des Quetschelements 6 und den geraden Einlaufabschnitt 15 für den Schlauch 3 trifft das Quetschelement 6 insofern sanft auf den Schlauch 3 auf, als es ihn zunächst zur wenig verformt, mit zunehmendem Bewegungsweg mehr und mehr in den Schlauch 3 eintaucht und diesen hierdurch stetig zunehmend bis zum in Figur 1 dargestellten vollständigen Abklemmen verformt.

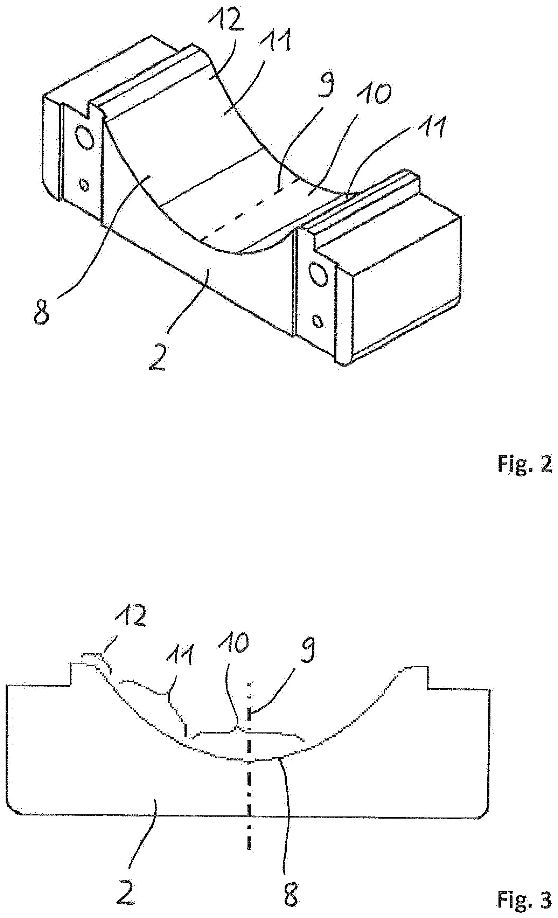

Die Figuren 2 und 3 zeigen einen Teil des Schlauchbetts 2 in einer perspektivischen Ansicht (Figur 2) und einer Seitenansicht (Figur 3). Erfindungsgemäß ist das Schlauchbett 2 mit einer PTFE-Beschichtung 8 versehen, die gegenüber dem Schlauch 3 reibungsvermindernd wirkt, so dass sich dieser beim Verformen geringfügig auf dem Schlauchbett 2 bewegen und unerwünschten plötzlichen Quetschungen ausweichen kann. Die Beschichtung 8 ist vorliegend ganzflächig auf das Schlauchbett 2 aufgebracht. Es ist im Rahmen der vorliegenden Erfindung jedoch auch denkbar, dass nur Teilbereiche des Schlauchbetts 2 mit einer reibungsvermindernden Beschichtung 8 versehen werden, um die erfindungsgemäßen Vorteile erzielen zu können.

Um dem Schlauch 3 Ausweichbewegungen auf dem Schlauchbett 2 zu gestatten, die unerwünschte Klemmungen sowie Druckimpulse vermindern, ist nicht nur die reibungsvermindernde Beschichtung 8 auf das Schlauchbett 2 aufgebracht worden, sondern dieses weist auch ein Profil auf, bei dem um eine Mitteilinie 9 ein mittlerer Bereich 10 mit einem Krümmungsradius vorgesehen ist, an den sich seitlich zwei Nachbarbereiche 11 mit abgeflachter Kontur anschließen; vorliegend weist diese Kontur ebenfalls einen (zweiten) Krümmungsradius auf, der jedoch größer ist als der erste Krümmungsradius des mittleren Bereichs 10. Außen an die Nachbarbereiche 11 schließen sich dann Randbereiche 12 an, die wiederum einen (dritten) Krümmungsradius aufweisen, der kleiner ist als der zweite Krümmungsradius der Nachbarbereiche 11 und vorliegend in etwa dem ersten Krümmungsradius des mittleren Bereichs 10 entspricht. Nach außen hin laufen die Randbereiche 12 in einer konvexen Krümmung aus.

Im vorliegenden Ausführungsbeispiel sorgt die Kombination des speziellen Profils des Schlauchbetts 2 mit der reibungsvermindernden Beschichtung 8 dafür, dass der Schlauch 3 beim Verformen durch das Quetschelement 6 schonender verformt wird als dies bisher im Stand der Technik möglich war, und insbesondere geringfügige Ausweichbewegungen in Längs- und Querrichtung ausführen kann, um ungewollten plötzlichen Quetschungen und Stauchungen auszuweichen und die Verformungsbewegung, die als Förderbewegung unverzichtbar ist, zu dämpfen. Diese Dämpfung sorgt schließlich dafür, dass die durch die Förderbewegung erzeugten Druckpulse sowie die aus Ladungstrennungen resultierenden elektrischen Spannungen im Schlauch signifikant vermindert werden.

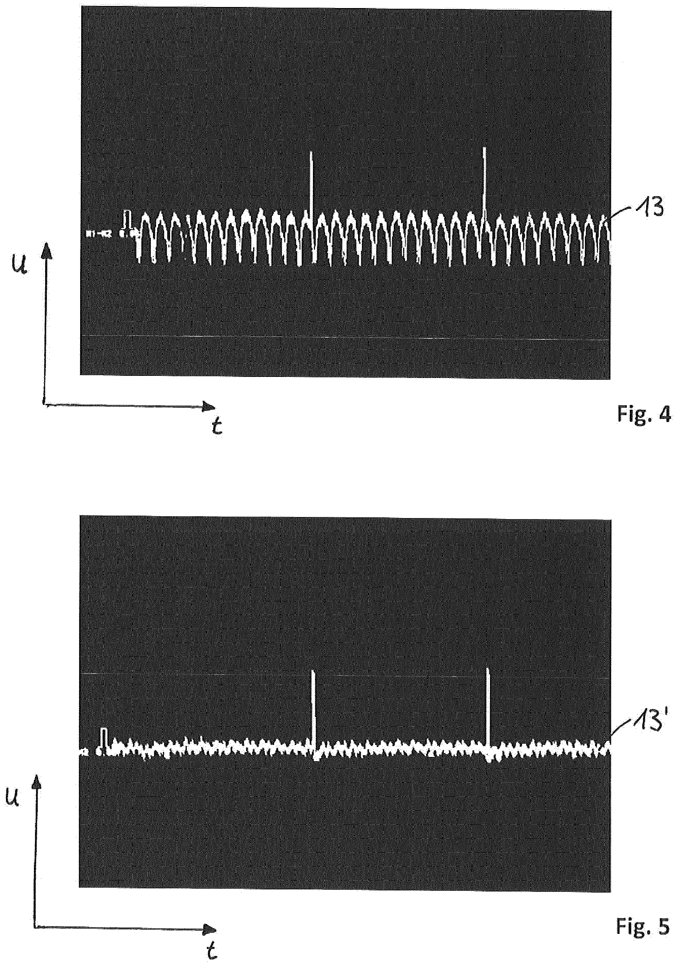

In den Figuren 4 und 5 sind zwei Messdiagramme dargestellt, deren Linien jeweils den Verlauf einer im Schlauch messbaren elektrischen Spannung über die Zeit aufgetragen zeigt. In Figur 4 handelt es sich um das Messdiagramm einer Schlauchpumpe, die gemäß dem Stand der Technik ausgestaltet ist, also ein Schlauchbett ohne reibungsvermindernde Beschichtung und ohne modifiziertes Profil enthält. Figur 5 zeigt das Messdiagramm einer erfindungsgemäß ausgestalteten Schlauchpumpe, also mit einem Schlauchbett mit modifiziertem Profil und reibungsvermindernden Beschichtung.

Das Spannungssignal 13 ist bei der Schlauchpumpe nach dem Stand der Technik (Figur 4) deutlich ausgeprägter als bei der erfindungsgemäß ausgestalteten Schlauchpumpe (Figur 5); die Maßstäbe der Diagramme aus den Figuren 4 und 5 sind identisch. Die Ausschläge des Spannungssignals 13' des Diagramms aus Figur 5 (erfindungsgemäße Schlauchpumpe) betragen beim vorliegend gewählten Messaufbau nicht einmal ein Viertel der Ausschläge des Spannungssignals 13 in Figur 4 (Stand der Technik). Wie anhand dieser Spannungssignale 13, 13', deutlich wird, wurde die im Schlauch messbare elektrische Spannung sehr deutlich (bis auf wenige Mikrovolt) reduziert. Dieser Unterschied kann entscheidend sein, um insbesondere ein EKG-Signal nicht nachteilig zu beeinflussen.

In den Spannungssignalen 13, 13' der Figuren 4 und 5 sind jeweils zwei Peaks zu erkennen. Hierbei handelt es sich um das Referenzsignal, das mit der im Schlauch messbaren elektrischen Spannung und deren erfindungsgemäßen Reduzierung nichts zu tun hat.

Da in medizintechnischen Anwendungen der behandelnde Arzt bei Patienten, deren Herzschlag mittels EKG überwacht wird und die gleichzeitig eine Infusion erhalten, die Form der EKG-Signale für seine Diagnose nutzt und auswertet, könnten Spannungssignale 13 sehr nachteilige Folgen haben, wenn sie sich dem EKG-Messsignal signifikant überlagen. Diese Gefahr ist mit der vorliegenden Erfindung gebannt.

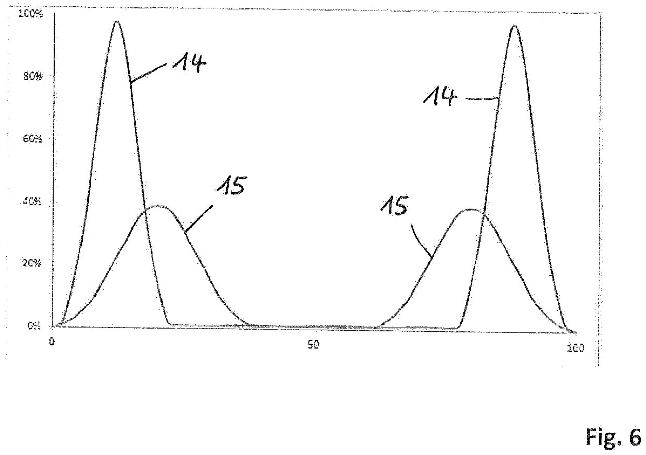

In Figur 6 sind die Änderungen der Verformung des Schlauchs einer Schlauchpumpe nach dem Stand der Technik und einer erfindungsgemäßen Schlauchpumpe in einem gemeinsamen Diagramm dargestellt. Hierzu ist die Ableitung der Verformungsbewegung des Schlauchs nach der Zeit über einen Pumpenzyklus hinweg aufgetragen.

Eine erste Kurve 14 zeigt die Verformungsänderungen des Schlauchs in einer Schlauchpumpe nach dem Stand der Technik. Es ist leicht erkennbar, dass der Schlauch mit steilen Flanken, also sehr abrupt und plötzlich verformt wird. Demgegenüber lässt sich einer zweiten Kurve 15, welche die Verformungsänderungen des Schlauchs in einer erfindungsgemäßen Schlauchpumpe zeigt, entnehmen, dass erfindungsgemäß eine sanftere Verformung des Schlauchs erfolgt, wobei die Ableitung der Verformungsbewegung nach der Zeit einen Maximalwert von 40% des Maximalwerts gemäß dem Stand der Technik nicht überschreitet.

## Patentansprüche

1. Schlauchpumpe, mit einem elastisch verformbaren Schlauch (3) für ein zu förderndes Medium (7) und mit einem Schlauchbett (2), über das der Schlauch (3) geführt ist, sowie mit mindestens einem entlang des Schlauchbetts (2) bewegbaren Quetschelement (6) zum Verformen des Schlauchs (3) auf dem Schlauchbett (2), um das Medium (7) hierdurch innerhalb des Schlauchs (3) vorwärtszubewegen,
**dadurch gekennzeichnet,**
**dass** das Schlauchbett (2) zumindest zum Teil mit einer reibungsvermindernden Beschichtung (8) versehen ist, und dass das Schlauchbett (2) ein Profil aufweist, das bezüglich einer in Längsrichtung des Schlauchs (3) verlaufenden Mittellinie (9) konkav geformt ist, wobei ein die Mittellinie (9) enthaltender mittlerer Bereich (10) einen ersten Krümmungsradius aufweist und zwei an den mittleren Bereich angrenzende Nachbarbereiche (11) zweite Krümmungsradien aufweisen, die größer als der erste Krümmungsradius sind.

2. Schlauchpumpe nach Anspruch 1,
**dadurch gekennzeichnet,**
**dass** die reibungsvermindernde Beschichtung (8) im Wesentlichen aus Kunststoffen, wie PTFE, und/oder aus Graphit, Diamant-Graphit, Parylenen und/oder einem harten Fett besteht.

3. Schlauchpumpe nach einem der Ansprüche 1 oder 2,
**dadurch gekennzeichnet,**
**dass** die reibungsvermindernde Beschichtung (8) fest am Schlauchbett (2) anhaftet.

4. Schlauchpumpe nach mindestens einem der Ansprüche 1 bis 3,
**dadurch gekennzeichnet,**
**dass** das Profil des Schlauchbetts (2) zwei Randbereiche (12) aufweist, die kleinere dritte Krümmungsradien als die zweiten Krümmungsradien der Nachbarbereiche (11) aufweisen.

5. Schlauchpumpe nach Anspruch 4,
**dadurch gekennzeichnet,**
**dass** die Randbereiche (12) des Profils mit einer konvexen Krümmung nach außen auslaufen.

6. Schlauchpumpe nach mindestens einem der Ansprüche 1 bis 5,
**dadurch gekennzeichnet,**
**dass** das Quetschelement (6) eine Rolle ist, die auf dem Schlauch (3) abrollt und diesen gegen das Schlauchbett (2) verformt.

7. Schlauchpumpe nach mindestens einem der Ansprüche 1 bis 6,
**dadurch gekennzeichnet,**
**dass** das Quetschelement (6) solcherart relativ zum Schlauchbett (2) geführt ist, dass es bei einer Bewegung entlang des Schlauchbetts (2) den Schlauch (3) zunächst nur wenig verformt und mit zunehmendem Bewegungsweg stetig zunehmend bis zum vollständigen Abklemmen verformt.

8. Schlauchpumpe nach mindestens einem der Ansprüche 1 bis 7,
**dadurch gekennzeichnet,**
**dass** der Schlauch (3) aus PVC, PTFE oder Silikon gefertigt ist.

## Claims

1. Peristaltic pump, having a resiliently deformable tube (3) for a medium (7) to be conveyed and having a tube bed (2) over which the tube (3) is guided, and having at least one squeezing element (6), which is movable along the tube bed (2), for deforming the tube (3) on the tube bed (2) in order thereby to move the medium (7) forwards inside the tube (3),
**characterised in that**
the tube bed (2) is provided at least in part with a friction-reducing coating (8), and the tube bed (2) has a profile which is concavely shaped in respect of a centre line (9) running in the longitudinal direction of the tube (3), wherein a central region (10) containing the centre line (9) has a first radius of curvature and two neighbouring regions (11) adjacent to the central region have second radii of curvature that are greater than the first radius of curvature.

2. Peristaltic pump according to claim 1,
**characterised in that**
the friction-reducing coating (8) consists substantially of plastics, such as PTFE, and/or of graphite, diamond-graphite, parylenes and/or a hard fat.

3. Peristaltic pump according to either one of claims 1 and 2,
**characterised in that**
the friction-reducing coating (8) adheres firmly to the tube bed (2).

4. Peristaltic pump according to at least one of claims 1 to 3,
**characterised in that**
the profile of the tube bed (2) has two edge regions (12) which have smaller third radii of curvature than the second radii of curvature of the neighbouring regions (11).

5. Peristaltic pump according to claim 4,
**characterised in that**
the edge regions (12) of the profile terminate towards the outside with convex curvature.

6. Peristaltic pump according to at least one of claims 1 to 5,
**characterised in that**
the squeezing element (6) is a roller which rolls on the tube (3) and deforms the tube against the tube bed (2).

7. Peristaltic pump according to at least one of claims 1 to 6,
**characterised in that**
the squeezing element (6) is guided relative to the tube bed (2) in such a way that on movement along the tube bed (2) it first deforms the tube (3) only slightly and with increasing movement along its path it deforms the tube at a constantly increasing rate until the tube is fully occluded.

8. Peristaltic pump according to at least one of claims 1 to 7,
**characterised in that**
the tube (3) is made from PVC, PTFE or silicone.

## Revendications

1. Pompe péristaltique comprenant un tuyau souple (3) élastiquement déformable, destiné à un fluide (7) devant être convoyé, et une niche (2) par l'intermédiaire de laquelle ledit tuyau souple (3) est guidé, ainsi qu'au moins un élément de compression (6) pouvant être mû le long de ladite niche (2) du tuyau souple et conçu pour déformer ledit tuyau souple (3) sur ladite niche (2), pour provoquer ainsi une progression dudit fluide (7) à l'intérieur dudit tuyau souple (3),
**caractérisée par le fait**
**que** la niche (2) du tuyau souple est dotée, au moins en partie, d'un revêtement (8) réducteur de frottement ; et par le fait que ladite niche (2) du tuyau souple offre un profil de configuration concave par rapport à une ligne médiane (9) s'étendant dans la direction longitudinale dudit tuyau souple (3), sachant qu'une région centrale (10), contenant ladite ligne médiane (9), présente un premier rayon de courbure et que deux régions attenantes (11), limitrophes de ladite région centrale, présentent des deuxièmes rayons de courbure supérieurs audit premier rayon de courbure.

2. Pompe péristaltique selon la revendication 1,
**caractérisée par le fait**
**que** le revêtement (8) réducteur de frottement consiste, pour l'essentiel, en des matières plastiques telles que le PTFE et/ou en du graphite, du graphite au diamant, des parylènes et/ou une graisse dure.

3. Pompe péristaltique selon l'une des revendications 1 ou 2,
**caractérisée par le fait**
**que** le revêtement (8) réducteur de frottement adhère fermement à la niche (2) du tuyau souple.

4. Pompe péristaltique selon au moins l'une des revendications 1 à 3,
**caractérisée par le fait**
**que** le profil de la niche (2) du tuyau souple comporte deux régions marginales (12), qui présentent des troisièmes rayons de courbure inférieurs aux deuxièmes rayons de courbure des régions attenantes (11).

5. Pompe péristaltique selon la revendication 4,
**caractérisée par le fait**
**que** les régions marginales (12) du profil s'achèvent, vers l'extérieur, par une courbure convexe.

6. Pompe péristaltique selon au moins l'une des revendications 1 à 5,
**caractérisée par le fait**
**que** l'élément de compression (6) est un rouleau qui roule sur le tuyau souple (3), et déforme ce dernier contre la niche (2) dudit tuyau souple.

7. Pompe péristaltique selon au moins l'une des revendications 1 à 6,
**caractérisée par le fait**
**que** l'élément de compression (6) est guidé, par rapport à la niche (2) du tuyau souple, de telle manière qu'il ne provoque, dans un premier temps, qu'une faible déformation dudit tuyau souple (3) lors d'un mouvement le long de ladite niche (2) et, au fur et à mesure de l'augmentation de la course dudit mouvement, une déformation à accroissement constant jusqu'au pincement intégral.

8. Pompe péristaltique selon au moins l'une des revendications 1 à 7,
**caractérisée par le fait**
**que** le tuyau souple (3) est fabriqué en PVC, en PTFE ou en silicone.
